# EUROPEAN PATENT SPECIFICATION

(11) **EP 1 295 894 B1**
(45) Date of publication and mention of the grant of the patent: **14.09.2011**
(21) Application number: 02021322.9
(22) Date of filing: 20.09.2002
(51) Int. Cl.: C07K 1/13

(54) **Method for an in vitro sequence specific biotinylation of polypeptides**
In vitro Verfahren zur sequenzspezifischen Biotinylierung von Polypeptiden
Procédé pour une biotinylation spécifique en séquence de polypeptides in vitro

(30) Priority: 25.09.2001 EP 01122554; 13.12.2001 EP 01129681
(43) Date of publication of application: 26.03.2003
(73) Proprietor: F. Hoffmann-La Roche AG, 4070 Basel (CH)
(72) Inventor: Ambrosius, Dorothee, 81477 München (DE); Lanzendoerfer, Martin, 81377 München (DE); Schraeml, Michael, 81375 München (DE); Watzele, Manfred, 82362 Weilheim (DE)
(74) Representative: Schreiner, Siegfried

(56) References cited:
- WO-A-95/04069
- CHAPMAN-SMITH A ET AL: "The enzymatic biotinylation of proteins: a post-translational modification of exceptional specificity" TIBS TRENDS IN BIOCHEMICAL SCIENCES, ELSEVIER PUBLICATION, CAMBRIDGE, EN, vol. 24, no. 9, 1 September 1999 (1999-09-01), pages 359-363, XP004178183 ISSN: 0968-0004

### SEQUENCE LISTING

<110> F. HOFFMANN-LA ROCHE AG
<120> Improved method for a sequence specific biotinylation <130> 20984
<140>
   <141>
<150> EP01122554.7
   <151> 2001-09-25
<150> EP01129681.1
   <151> 2001-12-13
<160> 13
<170> PatentIn Ver. 2.1
<210> 1
   <211> 123
   <212> PRT
   <213> Artificial Sequence
<220>
   <223> Description of Artificial Sequence:1.3S transcarboxylase subunit of Propionibacterium shermanii
<400> 1
   Met Lys Leu Lys Val Thr Val Asn Gly Thr Ala Tyr Asp Val Asp Val
    1               5                  10                  15
   Asp Val Asp Lys Ser His Glu Asn Pro Met Gly Thr Ile Leu Phe Gly
               20                  25                  30
   Gly Gly Thr Gly Gly Ala Pro Ala Pro Arg Ala Ala Gly Gly Ala Gly
           35                  40                  45
   Ala Gly Lys Ala Gly Glu Gly Glu Ile Pro Ala Pro Leu Ala Gly Thr
       50                  55                  60
   Val Ser Lys Ile Leu Val Lys Glu Gly Asp Thr Val Lys Ala Gly Gln
   65                  70                  75                  80
   Thr Val Leu Val Leu Glu Ala Met Lys Met Glu Thr Glu Ile Asn Ala
                   85                  90                  95
   Pro Thr Asp Gly Lys Val Glu Lys Val Leu Val Lys Glu Arg Asp Ala
               100                 105                 110
   Val Gln Gly Gly Gln Gly Leu Ile Lys Ile Gly
           115                 120
<210> 2
   <211> 156
   <212> PRT
   <213> Escherichia coli
<400> 2
   Met Asp Ile Arg Lys Ile Lys Lys Leu Ile Glu Leu Val Glu Glu Ser
   1 5 10 15
   Gly Ile Ser Glu Leu Glu Ile Ser Glu Gly Glu Glu Ser Val Arg Ile
   20 25 30
   Ser Arg Ala Ala Pro Ala Ala Ser Phe Pro Val Met Gln Gln Ala Tyr
   35 40 45
   Ala Ala Pro Met Met Gln Gln Pro Ala Gln Ser Asn Ala Ala Ala Pro
   50 55 60
   Ala Thr Val Pro Ser Met Glu Ala Pro Ala Ala Ala Glu Ile Ser Gly
   65 70 75 80
   His Ile Val Arg Ser Pro Met Val Gly Thr Phe Tyr Arg Thr Pro Ser
   85 90 95
   Pro Asp Ala Lys Ala Phe Ile Glu Val Gly Gln Lys Val Asn Val Gly
   100 105 110
   Asp Thr Leu Cys Ile Val Glu Ala Met Lys Met Met Asn Gln Ile Glu
   115 120 125
   Ala Asp Lys Ser Gly Thr Val Lys Ala Ile Leu Val Glu Ser Gly Gln
   130 135 140
   Pro Val Glu Phe Asp Glu Pro Leu Val Val Ile Glu
   145 150 155
<210> 3
   <211> 22
   <212> PRT
   <213> Artificial Sequence
<220>
   <223> Description of Artificial Sequence:Biotinylation peptide originating from the 1.3S transcarboxylase subunit of Propionibacterium shermanii
<400> 3
   Gly Gln Thr Val Leu Val Leu Glu Ala Met Lys Met Glu Thr Glu Ile
   1 5 10 15
   Asn Ala Pro Thr Asp Gly
   20
<210> 4
   <211> 21
   <212> PRT
   <213> Artificial Sequence
<220>
   <223> Description of Artificial Sequence:Biotinylation peptide AAW46671
<400> 4
   Asp Glu Glu Leu Asn Gln Ile Phe Glu Ala Met Lys Met Tyr Pro Leu
   1 5 10 15
   Val His Val Thr Lys
   20
<210> 5
   <211> 15
   <212> PRT
   <213> Artificial Sequence
<220>
   <223> Description of Artificial Sequence:Biotinylation peptide AAW46656
<400> 5
   Leu Leu Arg Thr Phe Glu Ala Met Lys Met Asp Trp Arg Asn Gly
   1 5 10 15
<210> 6
   <211> 15
   <212> PRT
   <213> Artificial Sequence
<220>
   <223> Description of Artificial Sequence:AviTag Biotinylation peptide
<400> 6
   Gly Leu Asn Asp Ile Phe Glu Ala Gln Lys Ile Glu Trp His Glu
   1 5 10 15
<210> 7
   <211> 133
   <212> PRT
   <213> Artificial Sequence
<220>
   <223> Description of Artificial Sequence:PinPoint Biotinylation peptide
<400> 7
   Met Lys Leu Lys Val Thr Val Asn Gly Thr Ala Tyr Asp Val Asp Val
   1               5                   10                  15
   Asp Val Asp Lys Ser His Glu Asn Pro Met Gly Thr Ile Leu Phe Gly
               20                  25                  30
   Gly Gly Thr Gly Gly Ala Pro Ala Pro Ala Ala Gly Gly Ala Gly Ala
           35                  40                  45
   Gly Lys Ala Gly Glu Gly Glu Ile Pro Ala Pro Leu Ala Gly Thr Val
       50                  55                  60
   Ser Lys Ile Leu Val Lys Glu Gly Asp Thr Val Lys Ala Gly Gln Thr
   65                  70                  75                  80
   Val Leu Val Leu Glu Ala Met Lys Met Glu Thr Glu Ile Asn Ala Pro
                   85                  90                  95
   Thr Asp Gly Lys Val Glu Lys Val Leu Val Lys Glu Arg Asp Ala Val
               100                 105                 110
   Gln Gly Gly Gln Gly Leu Ile Lys Ile Gly Asp Leu Glu Leu Ile Glu
           115                 120                 125
   Gly Arg Glu Lys Leu
       130
<210> 8
   <211> 39
   <212> DNA
   <213> Artificial Sequence
<220>
   <223> Description of Artificial Sequence:primer
<400> 8
   ataagaataa gcttcctgaa atctgcaaac aggatatcg 39
<210> 9
   <211> 35
   <212> DNA
   <213> Artificial Sequence
<220>
   <223> Description of Artificial Sequence:primer
<400> 9
   atagtttagc ggccgcttat cagcctagcc agtcg 35
<210> 10
   <211> 21
   <212> DNA
   <213> Artificial Sequence
<220>
   <223> Description of Artificial Sequence:primer
<400> 10
   gaaggcatat gggtctgaac g 21
<210> 11
   <211> 47
   <212> DNA
   <213> Artificial Sequence
<220>
   <223> Description of Artificial Sequence:primer
<400> 11
   ctcagaaaat cgaatggcac gaagcgaccc tgaaatctgc aaacagg 47
<210> 12
   <211> 48
   <212> DNA
   <213> Artificial Sequence
<220>
   <223> Description of Artificial Sequence:primer
<400> 12
   gccattcgat tttctgagct tcgaagatgt cgttcagacc catatgcc 48
<210> 13
   <211> 30
   <212> DNA
   <213> Artificial Sequence
<220>
   <223> Description of Artificial Sequence:primer
<400> 13
   gccgctcgag tcagcagcct agccagtcgg 30

## Description

The invention relates to an improved method for sequence specific biotinylation of polypeptides.

The enzyme biotin holoenzyme synthetase (BirA) of E.coli (a biotin ligase) catalyzes in vivo the covalent addition of biotin to the ε-amino group of a lysine side chain in its natural substrate, biotin carboxyl carrier protein (BCCP) (Cronan, J.E., Jr., et al., J. Biol. Chem. 265 (1990) 10327-10333). In E.coli only BCCP is biotinylated. This protein is a subunit of acetyl-CoA carboxylase. The reaction is catalyzed by the biotin-protein ligase BirA, the product of the qrA gene (Cronan, J.E., Jr., Cell 58 (1989) 427-429). Biotinylation of proteins using a biotinylation enzyme also by recombinant means is described in WO 95/04069:

Sequence specific enzymatic biotinylation using BirA is also described for recombinant polypeptides during expression in E.coli (Tsao, K.-L., et al., Gene 169 (1996) 59-64). Altman, J.D., et al., Science 274 (1996) 94-96, describe the enzymatic biotinylation of isolated polypeptides in vitro, using also BirA. Such a method is very laborious however, requiring considerably more purification steps compared to the biotinylation in vivo. First, the protein must be prepared, isolated and purified. Subsequently, biotinylation is performed, and thereafter, another purification is carried out. Parrott, M.B., and Barry, M.A., in Biochem. Biophys. Res. Communications 281 (2001) 993-1000, describe the metabolic biotinylation of secreted and cell-surface proteins from mammalian cells using the endogenous biotin ligase enzymes of the mammalian cell. Saviranta, P., et al., in Bioconjug. Chem. 9 (1998) 725-735, describe the in vitro enzymatic biotinylation of recombinant Fab fragments through a peptide acceptor tail. The proteins were recombinantly produced in E.coli, purified and subsequently biotinylated in vitro with BirA. After the removal of non-biotinylated Fab fragments, the overall yield of biotinylated Fab was 40%.

Both the in vitro as well as the in vivo biotinylation of heterologous polypeptides using biotin ligases such as BirA suffers from several drawbacks. Whereas in vitro biotinylation is very time-consuming and laborious, in vivo biotinylation results in products containing considerable amounts of BCCP. It is difficult to separate biotinylated BCCP from most of the desired biotinylated polypeptides, and it cannot be separated therefrom completely.

It was the object of the invention to provide an improved and simple method for the specific enzymatic biotinylation of polypeptides which provides specifically biotinylated polypeptides of high purity in a high yield and activity.

### Summary of the Invention

The invention provides a method for the synthesis of a biotinylated polypeptide characterized in that the synthesis is performed in a cell-free peptide synthesis reaction mixture containing a ribosome-containing cell lysate of a prokaryotic or eukaryotic cell by translation or transcription/translation of nucleic acids encoding the polypeptide and BirA, whereas the reaction mixture contains biotin. It was surprisingly found that the activity of polypeptides, biotinylated according to the method of the invention, was higher than the activity found for such polypeptides biotinylated in an in vivo cell fermentation system.

The invention therefore provides a method of preparing a biotinylated polypeptide in a cell-free peptide synthesis reaction mixture which contains ribosomes, tRNA, ATP, GTP, nucleotides, amino acids and BirA as a nucleic acid characterized in that
a) a nucleic acid is expressed to form said polypeptide which contains a holocarboxylase synthetase (BirA) substrate sequence tagged at either the N- or the C-terminus ; and BirA as nucleic acid is expressed in the reaction mixture to provide BirA polypeptide ;
b) said polypeptide is biotinylated in the presence of biotin and BirA;
c) said biotinylated polypeptide is isolated from said mixture; or said mixture is incubated with immobilized avidin or streptavidin under such conditions that said biotinylated polypeptide is bound to said immobilized avidin or streptavidin.

Preferably ribosomes are provided as cell lysate, preferably as E.coli lysate.

### Detailed Description of the Invention

The method according to the invention provides an improved method for the production of a polypeptide (polypeptide of interest) which is specifically biotinylated at an N- or C-terminal sequence tag by site-specific enzymatic biotinylation. Preferably, the polypeptide has a molecular weight of about 8 kDa to about 120 kDa, preferably a length of about 100 to 400 amino acids. According to the invention it is surprisingly possible to produce such biotinylated polypeptides without substantial contamination of biotin carboxyl carrier protein (BCCP) and without intermediate isolation of the non-biotinylated polypeptide if a cell-free peptide synthesis reaction mixture is used for the polypeptide synthesis.

A "cell-free peptide synthesis reaction mixture" according to the invention is described in the state of the art and is a cell-free lysate of prokaryotic or eukaryotic cells containing ribosomes, tRNA, ATP, GTP, nucleotides, and amino acids. A preferred prokaryote is E.coli.

Cell-free polypeptide synthesis is a method which has been known in the state of the art for a long time. Spirin et al. developed in 1988 a continuous-flow cell-free (CFCF) translation and coupled transcription/translation system in which a relatively high amount of protein synthesis occurs (Spirin, A.S., et al., Science 242 (1988) 1162-1164). For such cell-free protein synthesis, cell lysates containing ribosomes were used for translation or transcription/translation. Such cell-free extracts from E.coli were developed by, for example, Zubay, G., Ann. Rev. Genetics 7 (1973) 267-287 and were used by Pratt, J.M., et al., Nucleic Acids Research 9 (1981) 4459-4479 and Pratt et al., Transcription and Translation: A Practical Approach, Hames and Higgins (eds.), pp. 179-209, IRL Press, 1984. Further developments of the cell-free protein synthesis are described in US Patent No. 5,478,730; US Patent No. 5,571,690; EP 0 932 664; WO 99/50436; WO 00/58493; and WO 00/55353.

Eukaryotic cell-free expression systems are described by, for example, Skup, D., and Millward, S., Nucleic Acids Research 4 (1977) 3581-3587; Fresno, M., et al., Eur. J. Biochem. 68 (1976) 355-364; Pelham, H.R., and Jackson, R.J., Eur. J. Biochem. 67 (1976) 247-256; and in WO 98/31827.

Holocarboxylase synthetase (EC6.3.4.15, biotin protein ligase (BPL), BirA) is an enzyme responsible for the covalent attachment of biotin to the cognate protein. Biotin ligase is highly specific and reacts only on proteins showing a very high degree of conservation in the primary structure of the biotin attachment domain. This domain includes preferably the highly conserved AMKM tetrapeptide (Chapman-Smith, A., and Cronan, J.E., Jr., J. Nutr. 129, 2S Suppl., (1999) 477S-484S). Recombinant BirA enzyme is described in WO 99/37785.

BirA is added to the reaction mixture as a nucleic acid (in an expression vector, e.g. RNA, DNA) which is expressed (transcribed/translated) in the system like the protein of interest. The amount of nucleic acid depends on the expression rate of the used vector and the necessary amount of BirA enzyme in the reaction mixture. 1 ng of BirA plasmid DNA (e.g. on the basis of a commercially available E.coli expression vector such as pIVEX vectors, http://www.biochem.roche.com/RTS), or even less, is sufficient for a quantitative biotinylation reaction of proteins fused with a BirA biotinylation substrate peptide. The maximum yield of expressed and specifically biotinylated fusion protein is achieved, when the desired target protein encoding plasmid DNA is added at 10-15µg and the plasmid DNA, being responsible for the coexpression of BirA, is introduced with an amount between 1-10ng. The ratio of fusion protein encoding plasmid-DNA to BirA encoding plasmid DNA was found out to be optimal at a ratio of about 1500:1. It was found that the same level as above is sufficient for quantitative biotinylation of the expressed fusion protein. D(+)-biotin was added at 1 to 10 uM, preferably in about 2 µM to the reaction mixture.

The polypeptide of interest which has to be biotinylated specifically must contain, at the N-terminus or C-terminus, a sequence which is recognized by the biotin protein ligase (a BirA substrate sequence tag). As already mentioned, such sequences exhibit a common structure which preferably contains the amino acid motif AMKM. In addition, there exist further protein sequences which do not contain this consensus sequence but can also be biotinylated by biotin protein ligases (Schatz, P.J., Biotechnology 11 (1993) 1138-1143). Such biotinylation sequences usually have preferably a length of about less than 50 amino acids, most preferably a length of about 10 to 20 amino acids. Examples are described in US Patent No. 6,265,552, preferably the sequences 1-12 and 14-89 mentioned in this patent. Examples of polypeptide sequences which can be biotinylated enzymatically and site-specifically are also described in Cronan, J.E., Jr., et al., J. Biol. Chem. 265 (1990) 10327-10333; and Samols, D., et al., J. Biol. Chem. 263 (1988) 6461-6464, and examples are shown by SEQ ID NO:1 to SEQ ID NO:7. Further examples are shown in US Patent Nos. 5,723,584; 5,874,239; and 5,932,433.

After the expression of the fusion polypeptide in the cell-free system, biotinylation occurs under standard reaction conditions, preferably within 10 to 30 hours at 20°C to 36°C, most preferably at about 30°C, and the reaction mixture is preferably, after dialysis, for concentration and buffer exchange, centrifuged.

In a preferred embodiment of the invention, the solution is, due to its high purity, directly used for immobilization of the biotinylated polypeptide on surfaces which contain immobilized avidin or streptavidin (e.g. microtiter plates or biosensors) without further purification.

According to the invention it is possible to produce highly pure biotinylated polypeptides which can be bound to surfaces in ligand binding experiments, e.g. surface plasmon resonance spectroscopy or ELISA assays.

If required however, biotinylated polypeptides produced according to the present invention can be further purified under native conditions using matrices containing immobilized (preferably monomeric) avidin, streptavidin, or derivatives thereof. A variety of useful physically (Kohanski, R.A. and Lane, M.D. (1990) Methods Enzymol. 194-200), chemically (Morag, E., et al., Anal. Biochem. 243 (1996) 257-263) and genetically (Sano, T., and Cantor, C.R., Proc. Natl. Acad. Sci.USA 92 (1995) 3180-3184) modified forms of avidin or streptavidin have been described that still bind biotin specifically but with weaker affinity to facilitate a one step purification procedure.

The following examples, references, sequence listing and figures are provided to aid the understanding of the present invention, the true scope of which is set forth in the appended claims.

### Description of the Figures

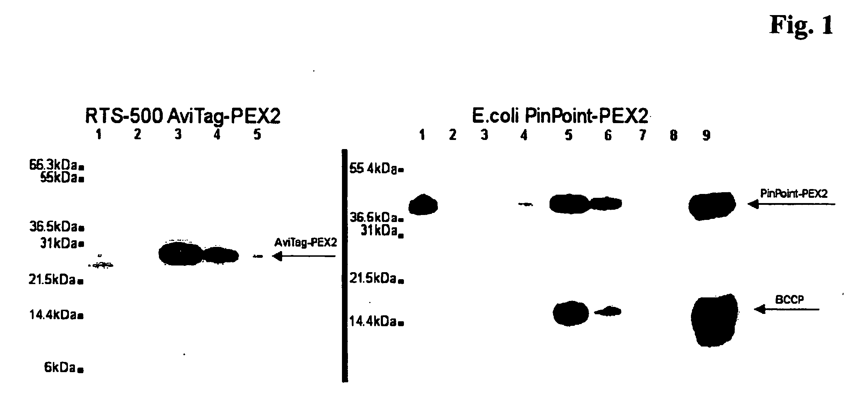

- Figure 1: Comparison of the biotinyl fusion proteins AviTag-PEX2 and PinPoint- PEX2. Two Western blots are shown, where biotinylated protein was detected with SA-POD conjugate in monomer avidin-sepharose elution fractions. Left panel, RTS 500 AviTag-PEX2: Lane 1: dialyzed and centrifuged supernatant of RTS 500 extract applied to the column. A second band under the target band indicates proteolytic degradation. Lane 2: column wash. Lanes 3,4 and 5: fractions of the 5mM biotin elution peak. Right panel, E.coli PinPoint-PEX2: Lane 1: centrifuged supernatant of E.coli cell lysate applied to the column. Lane 2: column wash. Lanes 3-8: fractions of the elution peak containing PinPoint-PEX2, whereby the co- concentration of a proteolytic degradation product of BCCP becomes obvious in lane 5 and lane 6 [3,16]. Lane 9: pooled elution fractions after ultrafiltration.

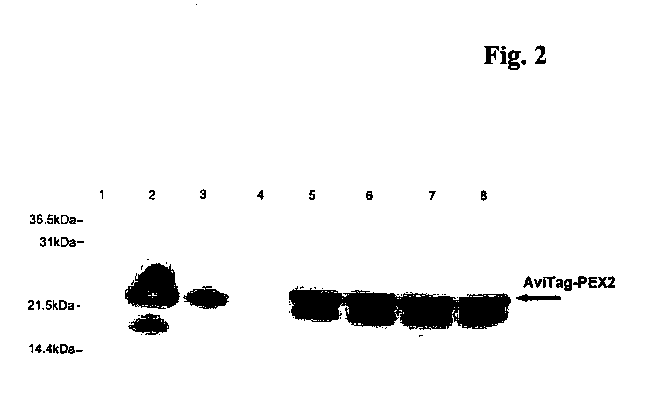

- Figure 2: Streptavidin-POD Westernblot showing biotinylated AviTag-PEX2 fusion protein.
The biotinylation reaction was done by coexpression of BirA. As a positive control chemically biotinylated PEX2 was used. Lanes 1-4 show the following:
[1] 10µg pIVEX2.1MCS AviTag PEX2, 2µM biotin, no pIVEX2.1MCSBirA addition
[2] 330ng chemically biotinylated PEX2, positive control
[3] 13ng chemically biotinylated PEX2, positive control
[4] 7ng chemically biotinylated PEX2, positive control not detectable
[5] 10µg pIVEX2.1MCS AviTag PEX2, 2µM biotin, 1µg pIVEX2.1MCSBirA
[6] 10µg pIVEX2.1MCS AviTag PEX2, 2µM biotin, 100ng pIVEX2.1MCSBirA
[7] 10µg pIVEX2.1MCS AviTag PEX2, 2µM biotin, 10ng pIVEX2.1MCSBirA
[8] 10µg pIVEX2.1MCS AviTag PEX2, 2µM biotin, 1ng pIVEX2.1MCSBirA

### Description of the Sequences

- **SEQ ID NO:1**: 1.3S transcarboxylase subunit of Propionibacterium shermanii
- **SEQ ID NO:2**: BCCP_E.coli
- **SEQ ID NO:3**: Biotinylation peptide originating from the 1.3S transcarboxylase subunit
- **SEQ ID NO:4**: Biotinylation peptide AAW46671
- **SEQ ID NO:5**: Biotinylation peptide AAW46656
- **SEQ ID NO:6**: AviTag Biotinylation peptide
- **SEQ ID NO:7**: PinPoint Biotinylation peptide
- **SEQ ID NO:8**: Primer
- **SEQ ID NO:9**: Primer
- **SEQ ID NO:10**: Primer
- **SEQ ID NO:11**: Primer
- **SEQ ID NO:12**: Primer
- **SEQ ID NO:13**: Primer

### Example 1

### Expression of PinPoint-PEX2 in vivo in E.coli (comparison)

PEX2 is the non-catalytic C-terminal hemopexin-like domain of MMP-2 (Brooks, P.C., et al., Cell 92 (1998) 391-400).

The gene, coding for PEX2 was amplified by PCR using the sense primer 5'-ATA AGA ATA AGC TTC CTG AAA TCT GCA AAC AGG ATA TCG-3' (SEQ ID NO:8) and antisense primer '5'-ATA GTT TAG CGG CCG CTT ATC AGC CTA GCC AGT CG-3' (SEQ ID NO:9). PCR was performed in 30 cycles with a temperature profile as follows: 1 min at 94°C, 1 min at 48°C and 1 min at 72°C. The PCR product was cloned as a NotI/HindIII fragment into an IPTG-inducible E.coli expression vector. The plasmid was transformed into an E.coli strain which contained the helper plasmid pUBS520 (Brinkmann, U., et al., Gene 85 (1989) 109-114). Cells were grown in LB-media containing 2 µM biotin, 100 µg/ml ampicillin and 50 µg/ml kanamycin. An overnight culture was used to inoculate 11 medium of the same composition, which was incubated under vigorous shaking at 37°C. At OD595 = 0.5, expression was induced with 1mM IPTG for 5 h. The cells (2.500 g) were harvested by centrifugation. The cell paste was resuspended (5 ml/g cell paste) in 50 mM TRIS pH 7.2, 20 mM NaCl, 5 mM CaCl2, 1 mg/ml lysozyme, Complete EDTA-Free protease inhibitor cocktail (Roche Diagnostics GmbH, Penzberg, Germany) and subsequently incubated for 20 min at room temperature. Further cell lysis was performed by sonication on ice until the suspension was no longer viscous. Crude lysate was centrifuged at 10,000 g for 30 min at 4°C and the supernatant was filtered through a 0.22 µm filter.

### Example 2

### Expression of AviTag-PEX2 in the cell-free expression system

The PEX2 gene was genetically fused with AviTag coding DNA by add-on PCR using the primers
5'-GAAGGCATATGGGTCTGAACG-3' (SEQ ID NO:10) (25 pmol),
5'-CTCAGAAAATCGAATGGCACGAAGCGACCCTGAAATCTGCAAACAGG-3' (SEQ ID NO:11) (10 pmol),
5'-GCCATTCGATTTTCTGAGCTTCGAAGATGTCGTTCAGACCCATATGCC-3' (SEQ ID NO:12) (10 pmol) and
5'-GCCGCTCGAGTCAGCAGCCTAGCCAGTCGG-3' (SEQ ID NO:13) (25 pmol).

The PCR program was performed as described above. The PCR product was digested with NdeI and XhoI and was cloned into an E.coli expression plasmid (pIVEX 2.3MCS plasmid of Roche Diagnostics GmbH, http://www.biochem.roche.com/RTS), previously cut with the same restriction enzymes. The plasmid was propagated in E.coli and isolated. 15 µg plasmid-DNA (ratio 260 nm/280 nm > 1.8) and 12.500 units biotin ligase holoenzyme (-2,5 ug biotin ligase, EC 6.3.4.15; Avidity Inc., Denver, USA) were added to the reaction mixture (1 ml) of a commercially available cell-free expression system (RTS 500, Roche Diagnostics GmbH, DE (http://www.biochem.roche.com/RTS)). Biotin ligase activity is defined by the manufacturer: 1 Unit is the amount of enzyme that will biotinylate 1 pmol of peptide substrate in 30 min at 30°C using the reaction mixture containing peptide substrate at 38 µM. The substrate used in the enzyme assay was a 15-mer variant of Sequence No. 85 as identified by Schatz, P.J., Biotechnology 11 (1993) 1138-1143. Biotin was adjusted to 2 µM in the reaction mixture and the feeding solution (12 ml). Protein expression was performed in the RTS 500 Incubator under stirring (130 rpm) for 17 h at 30°C. The product solution was subsequently dialyzed against buffer W2 (see Example 3) and centrifuged at 10,000 g for 30 min at 4°C.

The E.coli lysate was prepared according to Zubay G., Ann. Rev. Genetics 7 (1973) 267-287, and dialyzed against a buffer containing 100 mM HEPES-KOH pH 7.6/30°C, 14 mM magnesium acetate, 60 mM potassium acetate, 0.5 mM dithiothreitol. The lyophilized lysate was solubilized as recommended in the RTS500 system manual.

### Reaction mixture:

185 mM potassium acetate, 15 mM magnesium acetate, 4 % glycerol, 2.06 mM ATP, 1.02 mM CTP, 1.64 mM GTP, 1.02 mM UTP, 257 µM of each amino acid (all 20 naturally occurring amino acids), 10.8 µg/ml folic acid, 1.03 mM EDTA, 100 mM HEPES-KOH pH 7.6/30°C, 1 µg/ml rifampicin, 0.03 % sodium azide, 40 mM acetyl phosphate, 480 µg/ml tRNA from E. coli MRE600, 2 mM dithiothreitol, 10 mM mercaptoethane sulfonic acid, 70 mM KOH, 0.1 U/µl Rnase inhibitor, 15 µg/ml plasmid, 220 µl/ml E. coli A19 lysate, 2 U/µl T7-RNA polymerase.

### Feeding solution:

185 mM potassium acetate, 15 mM magnesium acetate, 4 % glycerol, 2.06 mM ATP, 1.02 mM CTP, 1.64 mM GTP, 1.02 mM UTP, 257 µM of each amino acid (all 20 naturally occurring amino acids), 10.8 µg/ml folic acid, 1.03 mM EDTA, 100 mM HEPES-KOH pH 7.5/30°C, 1 µg/ml rifampicin, 0.03 % sodium azide, 40 mM acetyl phosphate, 2 mM dithiothreitol, 10 mM mercaptoethane sulfonic acid, 70 mM KOH.

### Example 3

### Purification and quantification

### Purification of biotinylated fusion proteins:

1 ml monomeric avidin sepharose resin (SoftLink, Promega, Madison USA) was filled in a Pharmacia HR-5 column. After washing the column with 10 CV buffer W1 (50 mM TRIS pH 7.2, 20 mM NaCl) and 10 CV buffer W2 (W1 + 5 mM CaCl2), cell extract according to Example 1 or product solution according to Example 2 was applied with a flow rate of 0.1 ml/min. Washing with buffer W2 was done until no more protein was detectable in the flow-path of the column. To elute biotinylated protein, buffer W2 + 5 mM biotin was applied. The eluted protein peak was separated in 0.5 ml fractions. Fractions, containing biotinylated target protein, were pooled, free biotin was removed during ultrafiltration with buffer W2.

### Detection and quantification of the fusion proteins:

The soluble and insoluble protein fractions were resolved by SDS-PAGE (10% BIS-TRIS SDS-polyacrylamide gel) and either stained with Coomassie brilliant blue or transferred to a PVDF-membrane by using the semy-dry Multiphor II apparatus (Pharmacia Biotech, Uppsala, Sweden) for 70 min at 120 V and room temperature. After the transfer was completed, the membrane was blocked in PBS plus 0.2% Tween 20 (PBS-Tween) and 5% (w/v) dry milk powder with gentle agitation at 4°C. PEX2 bound to the PVDF-membrane was detected with a PEX2-specific antibody. The antibody stock solution was 1.47 mg/ml polyclonal rabbit anti-PEX2-IgG, directed against the whole molecule. The membrane was incubated for 1 hour at room temperature in PBS-Tween, 2.5% (w/v) dry milk powder, containing PEX2 antiserum (1 : 50.000 v/v) followed by three ten minute washes. The membrane was incubated for 1hr in PBS-Tween+2.5% (w/v)dry milk powder with 1 : 50,000 anti-mouse/anti-rabbit-IgG-POD conjugate (Roche Diagnostics GmbH, DE) followed by three ten minute washes in PBS-Tween. The Western blot was developed with the Chemiluminescence Western Blotting Kit (Mouse/Rabbit, Roche Diagnostics GmbH, DE).

After the densitometric detection of PEX2 protein, the membrane was regenerated for 10 min in 0.1M NaOH and subsequently washed 3 x 10 min in PBS-Tween. The membrane was blocked and washed again as described above. Detection of biotinylated fusion protein was carried out by incubating the regenerated membrane in a 1 : 4000 (v/v) dilution of streptavidin-POD conjugate (Roche Diagnostics GmbH, DE) in PBS-Tween buffer +2.5% (w/v) dry milk powder, for 1hour. After washing the membrane three times for 10 min with PBS-Tween, the Western blot was developed again. Biotinylation levels of the PEX2 fusion proteins were determined by comparison of densitometric data of the two detection steps.

Densitometric quantification of detected protein bands was performed by calibration using verified quantities of recombinant, chemically biotinylated PEX2 and the software ImageMaster 1D Prime ID Elite (Amersham Pharmacia Biotech Europe GmbH, Freiburg, DE).

### Plasmon Resonance Spectroscopy:

Activity of the biotinylated PEX2 fusion proteins was measured using plasmon spectroscopy (BIacore 3000 technology, BIAcore AB, Uppsala, SE). The system was running under HBS-P-buffer. PEX2 fusion proteins were immobilized on streptavidin coated BIAcore-SA chips in a manner as recommended by the manufacturer. Various dilution steps of a 200 nM TIMP2 (Yu, A.E., et al., Biochem. Cell Biol. 74 (1996) 823-831) stock solution (0.33 mg/ml in 1 x PBS-buffer) in HBS-P buffer were used to measure kinetic data in accordance to the manufacturers instructions. TIMP2 was eluted from the chip with ImmunoPure Gentle Ag/Ab Elution buffer (PIERCE).

### Example 4

### Investigation of purity

### a) Biotinylated PinPoint-PEX2 in E.coli (comparison)

PEX2, N-terminally fused with the PinPoint-tag, was expressed and specifically biotinylated in vivo in E.coli in accordance with Example 1. The fusion protein has a calculated molecular mass of 36 kDa. Protein identity was confirmed by N-terminal Edman degradation. Harvesting of 11 fermentation culture resulted in 6 gram bacterial wet mass. A total yield of 0.4 mg fusion protein per gram cell paste was determined by densitometric quantification of Western blots performed using PEX2-specific antibodies. Approximately 10% of the target protein was enriched in the supernatant of the cleared cell lysate. Quantification of biotinylation yield was determined by comparing densitometric data as described in material and methods. Using streptavidin-POD (peroxidase) conjugate in a colorimetric assay, no other biotinylated protein could be detected in the crude cell lysate, whereas monomeric avidin affinity chromatography enriched a second biotinylated protein in the elution fractions. Further analysis of the eluate using streptavidin-POD conjugate revealed two protein bands. The first band with an approximate mass of 40 kDa was the desired biotinylated fusion protein PinPoint-PEX2. The second protein with a size of approximately 16 kDa is BCCP, the only biotinylated protein found in E.coli. Contamination with this second biotinylated protein accounted for up to 50% of the total yield. The PinPoint-PEX2 containing elution fractions were pooled. The excess of free biotin was removed via ultrafiltration. Two samples with different degree of purity were analyzed in surface plasmon resonance spectroscopy using BIAcore technology. The activity of an immobilized ligand is indicated by the maximum analyte binding capacity. First, activity of the partially purified protein concentrate was analyzed. 380 RU of PinPoint-PEX2 (ligand) were immobilized on a BIAcore SA-chip. Saturation of the protein ligand on the chips surface with TIMP2 (analyte) was reached at Rmax=61RU. Based on this data, a ligand binding activity of 26% was calculated. In a second setting, 664RU of biotinyl-protein were immobilized by injecting supernatant of dialyzed and cleared cell lysate in the flow-cell. Saturation with the analyte TIMP2 was reached at 61 RUmax and the calculated ligand binding activity was 15%. In both cases kinetic data of the TIMP2/PinPoint-PEX2 interaction were determined. An equilibrium constant of KD = 1.5x10⁻¹⁰ M was calculated using a numeric Langmuir simulation model of a binary complex formation.

### b) Biotinylated AviTag-PEX2 (invention)

PEX2, N-terminally fused with the AviTag biotin-acceptor sequence, was expressed and biotinylated in vitro in the RTS 500 in accordance with Example 2. Biotinylation was facilitated by adding 12.500 units of BirA-enzyme to the reaction mix. The expressed fusion protein has a molecular mass of 25 kDa and was detected by Western blotting, using the PEX2-specific antibody and streptavidin-POD conjugate. When compared to a molecular weight standard, the fusion protein shows an apparent mass of 25 kDa in a 10% Bis-Tris SDS-PAGE. Densitometric quantification showed a total yield of 72 µg AviTag-PEX2 per milliliter of RTS 500 extract. The proportion of soluble fusion protein was 50% of the total yield. The degree of biotinylation was analyzed as described in material and methods and found to be quantitative. Detection of biotinylated protein with streptavidin-POD conjugate showed no other biotinylated protein in the extract. After the affinity purification procedure using monomeric avidin, only biotinylated AviTag-PEX2 fusion protein was detected in the elution fractions. The identity of the fusion protein was confirmed by N-terminal degradation (Edman). Purified AviTag-PEX2 fusion protein as well as supernatant from dialyzed and cleared RTS 500 extract were analyzed in surface plasmon resonance spectroscopy. 105 RU purified AviTag-PEX2 fusion protein were attached to a BIAcore SA-chip. Saturation of the immobilized AviTag-PEX2 ligand with the analyte TIMP2 was achieved at 64 RUmax. Thus, an analyte binding capacity of 70% (compared to 26% according to Comparison Example 4a) could be detected. After the injection of cleared supernatant of RTS 500 extract, 732 RU biotinylated protein were immobilized on the SA-chips surface. At Rmax, 341RU TIMP2 were bound, which resembles an analyte binding capacity of 53% (compared to 15% according to Comparison Example 4a). Kinetics were measured showing an equilibrium constant of the TIMP2 to PEX2 interaction of KD =1.5x10⁻¹⁰ M. The KD was determined using the numeric model described before.

### Example 5

### Biotinylation of AviTag-PEX2 by coexpression of BirA in the RTS 500

### Material and Methods:

Five RTS 500 reactions were prepared according to the manufacturer's instructions. D-Biotin was adjusted to 2µM in each reaction. 10µg pIVEX2.3MCS containing DNA encoding AviTag-PEX2 (ratio 260nm/280nm > 1.8 ) was added to each reaction mixture. Instead of a supplementation with recombinant BirA, as described in Example 2, pIVEX2.3MCS containing DNA encoding BirA was added at varying amounts (1µg, 100ng, 10ng, 1ng, 0ng, ratio 260nm/280nm >1.8) to the reaction chambers. During protein expression the fusion protein AviTag-PEX2 and BirA were simultaneously expressed. The coexpression was performed (Roche Diagnostics GmbH) under stirring (130rpm) for 17h at 30°C. The RTS lysates were centrifuged at 10.000g for 10min. The supernatant of each reaction was analyzed for biotinylated AviTag-PEX2 fusion protein using streptavidin POD Western blotting as described in Example 3.

### Results:

Without any supplementation of pIVEX2.3MCSBirA plasmid-DNA no biotinylated AviTag-PEX2 fusion protein could be detected in streptavidin POD Western blotting (Fig. 2, lane[1]), whereas addition of pIVEX2.3MCSBirA showed a biotinylated AviTag-PEX2 product (lanes [5,6,7,8]). 1ng of pIVEX2.3MCSBirA plasmid-DNA inserted into the system is enough plasmid DNA to coexpress sufficient amounts of active BirA, in order to quantitatively biotinylate AviTag-PEX2 fusion protein.

### List of References

Altman, J.D., et al., Science 274 (1996) 94-96
Brinkmann, U., et al., Gene 85 (1989) 109-114
Brooks, P.C., et al., Cell 92 (1998) 391-400
Chapman-Smith, A., and Cronan, J.E., Jr., J. Nutr. 129, 2S Suppl., (1999) 477S-484S
Cronan, J.E., Jr., Cell 58 (1989) 427-429
Cronan, J.E., Jr., et al., J. Biol. Chem. 265 (1990) 10327-10333
EP 0 932 664
Fresno, M., et al., Eur. J. Biochem. 68 (1976) 355-364
Kohanski, R.A. and Lane, M.D. (1990) Methods Enzymol. 194-200
Morag, E., et al., Anal. Biochem. 243 (1996) 257-263
Parrott, M.B., and Barry, M.A., Biochem. Biophys. Res. Communications 281 (2001) 993-1000
Pelham, H.R., and Jackson, R.J., Eur. J. Biochem. 67 (1976) 247-256
Pratt et al., Transcription and Translation: A Practical Approach, Hames and Higgins (eds.), pp. 179-209, IRL Press, 1984
Pratt, J.M., et al., Nucleic Acids Research 9 (1981) 4459-4479
Samols, D., et al., J. Biol. Chem. 263 (1988) 6461-6464
Sano, T., and Cantor, C.R., Proc. Natl. Acad. Sci.USA 92 (1995) 3180-3184
Saviranta, P., et al., Bioconjug. Chem. 9 (1998) 725-735
Schatz, P.J., Biotechnology 11 (1993) 1138-1143
Skup, D., and Millward, S., Nucleic Acids Research 4 (1977) 3581-3587
Spirin, A.S., et al., Science 242 (1988) 1162-1164
Tsao, K.-L., et al., Gene 169 (1996) 59-64
US Patent No. 5,478,730
US Patent No. 5,571,690
US Patent No. 5,723,584
US Patent No. 5,874,239
US Patent No. 5,932,433
US Patent No. 6,265,552
WO 00/55353
WO 00/58493
WO 95/04069
WO 98/31827
WO 99/37785
WO 99/50436
Yu, A.E., et al., Biochem. Cell Biol. 74 (1996) 823-831
Zubay, G., Ann. Rev. Genetics 7 (1973) 267-287

## Claims

1. A method of preparing a biotinylated polypeptide in a cell-free peptide synthesis reaction mixture which contains ribosomes, tRNA, ATP, GTP, nucleotides and amino acids and BirA as a nucleic acid, **characterized in that**
a) a nucleic acid is expressed to form said polypeptide which contains a holocarboxylase synthetase (BirA) substrate sequence tagged at either the N-terminus or C-terminus; and BirA as a nucleic acid is expressed in said reaction mixture to provide BirA polypeptide;
b) said polypeptide is biotinylated in the presence of biotin and BirA;
c) said biotinylated polypeptide is isolated from said mixture; or said mixture is incubated with immobilized avidin or streptavidin under such conditions that said biotinylated polypeptide is bound to said immobilized avidin or streptavidin.

2. A method according to claim 1, **characterized in that** ratio of polypeptide encoding DNA to BirA encoding DNA is 1500:1.

## Patentansprüche

1. Verfahren zur Herstellung eines biotinylierten Polypeptids in einer zellfreien Peptidsynthese-Reaktionsmischung, die Ribosomen, tRNA, ATP, GTP, Nukleotide und Aminosäuren und BirA als Nukleinsäure enthält, **dadurch gekennzeichnet, dass**
a) eine Nukleinsäure exprimiert wird, um das Polypeptid zu bilden, welches eine Holocarboxylasesynthetase- (BirA) Substratsequenz enthält, mit der entweder der N-Terminus oder der C-Terminus markiert ist; und wobei BirA als Nucleinsäure in der Reaktionsmischung exprimiert wird, um BirA-Polypeptid bereitzustellen;
b) das Polypeptid in Anwesenheit von Biotin und BirA biotinyliert wird;
c) das biotinylierte Polypeptid aus der Mischung isoliert wird; oder die Mischung mit immobilisiertem Avidin oder Streptavidin unter solchen Bedingungen inkubiert wird, dass das biotinylierte Polypeptid an das immobilisierte Avidin oder Streptavidin gebunden wird.

2. Verfahren nach Anspruch 1, **dadurch gekennzeichnet, dass** das Verhältnis von Polypeptid-kodierender DNA zu BirA-kodierender DNA 1500:1 ist.

## Revendications

1. Procédé de préparation d'un polypeptide biotinylé dans un mélange réactionnel de synthèse de peptides sans cellules contenant des ribosomes de l'ARNt, de l'ATP, du GTP, des nucléotides et des acides aminés et BirA en tant qu'acide nucléique, **caractérisé en ce que**
a) on fait s'exprimer un acide nucléique pour former ledit polypeptide qui contient une séquence de substrat d'holocarboxylase synthétase, (BirA) étiquetée à l'une ou l'autre des extrémités N-terminale et C-terminale; et on fait s'exprimer BirA en tant qu'acide nucléique dans ledit mélange réactionnnel pour fournir le polypeptide BirA;
b) on biotinyle ledit polypeptide en présence de biotine et de BirA;
c) on isole ledit polypeptide biotinylé dudit mélange; ou on met ledit mélange à incuber avec de l'avidine ou de la streptavidine immobilisée dans des conditions telles que ledit polypeptide biotinylé se lie à ladite avidine ou streptavidine immobilisée.

2. Procédé selon la revendication 1, **caractérisé en ce que** le rapport de l'ADN codant pour le polypeptide à l'ADN codant pour BirA est de 15000:1.
